Europäisches Patentamt

European Patent Office

Office européen des brevets

(19)

(11) Veröffentlichungsnummer: **0 091 611**
A1

(12) # EUROPÄISCHE PATENTANMELDUNG

(21) Anmeldenummer: 83103174.5

(22) Anmeldetag: 30.03.83

(51) Int. Cl.³: **A 23 G 3/30**
**A 61 K 7/16**

(30) Priorität: 08.04.82 DE 3213284

(43) Veröffentlichungstag der Anmeldung:
19.10.83 Patentblatt 83/42

(84) Benannte Vertragsstaaten:
AT BE CH DE FR GB IT LI LU NL SE

(71) Anmelder: Kruppa, Winfried
Vilstalstrasse 88
D-8451 Kümmersbruck(DE)

(72) Erfinder: Kruppa, Winfried
Vilstalstrasse 88
D-8451 Kümmersbruck(DE)

(74) Vertreter: Reinhard, Kreutz & Skuhra
Leopoldstrasse 51
D-8000 München 40(DE)

(54) Antikaries-Kaugummi.

(57) Es wird ein neuer Antikaries-Kaugummi offenbart, der eine an sich bekannte Kaugummi-Grundmasse, Natriumfluorid, Calciumcitrat, Tricalcium-bis-Orthophosphat und Zusatzstoffe enthält und dadurch gekennzeichnet ist, daß er Zein G-200 enthält. Zein G-200 ist ein Proteinprodukt (Prolamin), das durch Extraktion mit verdünntem Ethylalkohol aus dem Kleber von Maissamen gewonnen wird. Es ist ein Copolymeres von Aminosäuren mit einem Molekulargewicht von ca. 25 000, das in wäßrigen Alkoholen, in Glykolen und Glykolethern und in Alkalien, nicht aber in Wasser löslich ist. Weiterhin wird ein Verfahren zur Herstellung des Antikaries-Kaugummi offenbart, bei welchem zunächst Calciumcitrat und Tricalcium-bis-Orthophosphat mit der Grundmasse durch Kneten vermischt werden und zu dieser Mischung dann das mit Zein G-200 ummantelte NaF in saurem Milieu zusammen mit den üblichen Zusatzstoffen als homogene Emulsion mittels eines Homogenisators eingebracht wird.

EP 0 091 611 A1

-1-

## Antikaries-Kaugummi

Die vorliegende Erfindung betrifft einen Antikaries-Kaugummi, enthaltend eine an sich bekannte Kaugummi-Grundmasse, Natriumfluorid, Calciumcitrat, Tricalcium-bis-Orthophosphat und Zusatzstoffe sowie ein Verfahren zu seiner Herstellung.

98 % aller Deutschen leiden seit ihrer Kindheit unter Karies. Es ist bekannt, zur Kariesprophylaxe Fluor bzw. Fluoride einzusetzen. Dazu kommt eine Zufuhr von Mineralstoffen, insbesondere von Calcium und Phosphaten. Diese können in Zahncremes, Mundwässern, Tabletten, Gelen und Lacken angewendet werden.

Die Erfahrung hat jedoch gezeigt, daß mit diesen Anwendungsmethoden keine befriedigenden Ergebnisse erzielbar sind, insbesondere, weil diese nicht regelmäßig angewendet werden können.

Andererseits sind bereits Kaugummis bekannt. Diese enthalten als Hauptbestandteil Chicle-Gummi oder andere Grundmassen, wie Butadien-Styrol-Copolymerisate, Gutta, Polyäthylen, Polyvinylester etc., denen Zusatzstoffe zugesetzt werden können, und zwar gemäß der Kaugummi-Verordnung vom 19.12.1959 (BGBl. I S. 754) und Änderungs-Verordnung vom 21.08.1964 (BGBl. I S. 703).

Eine Aufgabe der vorliegenden Erfindung ist es, die mit der vorstehend geschilderten Anwendung von Fluor und Mineralstoffen verbundenen Nachteile zu vermeiden, insbesondere eine Möglichkeit zu schaffen, Fluor und Mineralstoffe ohne schädigende Nebenwirkungen auf Dauer anwenden zu können.

Diese Aufgabe wird erfindungsgemäß dadurch gelöst, daß der Antikaries-Kaugummi der eingangs beschriebenen Art Zein G-200 enthält.

Erfindungsgemäß ist das in dem Kaugummi enthaltende Natriumfluorid mit 0,0004 bis 0,001 Gew.% Zein G-200 ummantelt.

In einer vorzugsweisen Ausführungsform besteht der erfindungsgemäße Antikaries-Kaugummi, bezogen auf den gesamten Kaugummi = 100 Gew.%, aus
0,04 bis 0,05 Gew.% Natriumfluorid,
0,0004 bis 0,001 Gew.% Zein G-200,
12 Gew.% Calciumcitrat,
4 Gew.% Tricalcium-bis-Orthophosphat und
Rest Kaugummi-Grundmasse.

Das erfindungsgemäße Verfahren zur Herstellung des Antikaries-Kaugummis ist dadurch gekennzeichnet, daß zunächst Calciumcitrat und Tricalcium-bis-Orthophosphat mit

-3-

der Grundmasse durch Kneten vermischt werden und daß zu dieser Mischung dann das mit Zein G-200 ummantelte NaF in saurem Milieu zusammen mit den üblichen Zusatzstoffen als homogene Emulsion mittels eines Homogenisators eingebracht wird. Zein G-200 ist ein Proteinprodukt (Prolamin), das durch Extraktion mit verdünntem Ethylalkohol aus dem Kleber von Maissamen gewonnen wird. Es ist ein Copolymeres von Aminosäuren mit einem Molekulargewicht von ca. 25 000, das in wäßrigen Alkoholen, in Glykolen und Glykolethern und in Alkalien, nicht aber in Wasser löslich ist. Eine noch genauere Definition findet sich im Lexikon der Hilfsstoffe von H.P.Fiedler, Ausgabe 1981, 2. Band, Seite 1020, Editio Cantor Verlag, Aulendorf. Diese genauere Definition wird im folgenden wiedergegeben:

Zein G-200 ist ein Proteinprodukt (Prolamin), das durch Extraktion mit verdünntem Ethylalkohol aus dem Kleber von Maissamen gewonnen wird. Es ist ein Copolymeres von Aminosäuren mit einem Molekulargewicht von ca. 25 000, das in wässrigen Alkoholen, in Glykolen und Glykolethern und in Alkalien, nicht aber in Wasser löslich ist. Zein G-200 ist ein leicht cremefarbiges, amorphes Pulver, für das folgende Kennzahlen angegeben werden: $D.^{25}$ 1,25, F. (Zersetzung) 180-200$^{\circ}$, isoelektrischer Punkt 6,2, Dielektrizitätskonstante 4,9-5,0. Es besteht mindestens zu 98 % aus Protein und enthält maximal 8 % flüchtige Anteile. Zusammensetzung (in %, die in Klammern angegebenen Zahlen geben Mol-Werte/Mol Zein (Molekulargewicht 25 000) an): Glutamin 21,4 (43), Glutaminsäure 1,5 (3), Leucin 19,3 (44), Isoleucin 6,2 (14), Prolin 9,0 (24), Alanin 8,3 (30), Phenylalanin 6,8 (12), Tyrosin 5,1 (8), Threonin 2,7 (7), Valin 3,1 (8), Serin 5,7 (17), Asparagin 4,5 (10), Methionin 2,0 (4), Arginin 1,8 (3), Histidin 1,1 (2), Cystin 0,8 (1), Glycin 0,7 (3). Reaktionsfähige Gruppen: Amino-1, Amid-53, Hydroxyl-24, Carboxyl-4 und Phenol-8.

Im folgenden wird ein Beispiel für die Erfindung gegeben, das die beste Ausführung darstellt:

Nach dem vorstehenden Verfahren wurden 1,25 g Kaugummi (=100 Gew.%) hergestellt. Zunächst wurden 0,15 g Calciumcitrat und 0,05 g Tricalcium-bis-Orthophosphat mit der Grundmasse durch Kneten vermischt. Sodann wurden 553 mg Natriumfluorid mit 5 bis 6 mg Zein G-200 ummantelt und in saurem Milieu zusammen mit den üblichen Zusatzstoffen als homogene Emulsion mittels eines Homogenisators eingebracht.

Das Ummanteln von NaF mit Zein G-200 erfolgt nach einem Besprühverfahren im Wirbelgegenstrom, auch Wirbelschichtgranulation oder Wirbelstromverfahren im Glattsystem genannt. Das NaF wird in einem geschlossenen Kessel von unten eingeblasen. Von oben und von den Seiten wird durch Düsen eine ca. 2,5 prozentige Zein/Äthylalkohollösung eingesprüht. Durch anschließend eingeblasene Warmluft wird im Wirbelstrom der Äthylalkohol wieder verdunstet und abgesaugt. Bei der Trocknung kommt es zur Mikroummantelung.

Die Ph-Wert-Einstellung bei der Einbringung des mit Zein ummantelten NaF zusammen mit den ätherischen Ölen erfolgt vorzugsweise mit Zitronensäure. Damit sich die Ummantelung des NaF nicht schon bei der Herstellung während der Vermengung mit den übrigen Inhaltsstoffen auflöst, wird das mit Zein G-200 ummantelte NaF in eine vorzugsweise mit Zitronensäure auf pH 1,5 bis 1,8 eingestellte wässrige Lösung eingebracht und bei einer Temperatur von ca. 25 bis 30° einige Stunden stehengelassen. Dabei lagert sich in die äußerste Schicht des Zein die Säure an. Anschließend wird mit Hilfe eines Emulgators das so vorbehandelte NaF mit den ätherischen Ölen zu einer Emulsion verarbeitet. Das ätherische Öl legt sich wie ein Mantel um das vorbehandelte NaF, so daß sich zwischen dem ätherischen Öl und der Zeinschicht ein Säurepuffer bildet, das auch bei der weiteren Einarbeitung in die Kaugummimasse erhalten bleibt und sich erst beim Kauvorgang in der Mundhöhle durch die dort bestehenden Bedingungen, insbesondere Enzyme und überwiegendes basisches Milieu der übrigen Inhaltsstoffe auflöst, so daß das NaF in der Mundhöhle freigesetzt wird. Nicht freigesetztes NaF würde in der Ummantelung behalten oder würde im Magen bei einem pH-Wert von 1 nicht gelöst werden und ungelöst aus dem Körper ausgeschieden werden. Daher besteht keine Gefahr, daß sich Fluor in den Knochen ablagert.

Mit dem erfindungsgemäßen Antikaries-Kaugummi wird verhindert, daß sich Fluor in der Knochensubstanz ablagert. Das mit Zein ummantelte Natriumfluorid kann nämlich nur in der Mundhöhle gelöst werden. Der pH-Wert der Mundhöhle beträgt normalerweise 7 bis 7,5. Beim Kauen mit dem erfindungsgemäßen Antikaries-Kaugummi steigt der pH-Wert von 8 bis 9. Gelangt ungelöstes, mit Zein ummanteltes Natriumfluorid in den Magen, wird beim dort bestehenden pH-Wert von 1 nicht gelöst und wird aus dem Körper ungelöst ausgeschieden.

Der vorzugsweise in Form von Dragees hergestellte erfindungsgemäße Antikaries-Kaugummi wird von allen Altersstufen, besonders aber von Kindern, bei denen die Kariesprophylaxe und Kariesreduzierung besonders vorrangig ist, gerne genommen.

Physiologisch gesehen ist Fluor ein Spurenelement. Die täglich mit der Nahrung zugeführte Fluormenge beträgt ca. 0,3 mg. Ähnliches trifft für Calcium und Phosphat zu. Erst durch eine höhere Dosierung werden diese Wirkstoffe zum Arzneistoff. Durch den erfindungsgemäßen Antikaries-Kaugummi können die erforderlichen Mengen an Wirkstoffen bzw. Arzneistoffen zugeführt werden.

Patentansprüche

1. Antikaries-Kaugummi, enthaltend eine an sich bekannte Kaugummi-Grundmasse, Natriumfluorid, Calciumcitrat, Tricalcium-bis-Orthophosphat und Zusatzstoffe, dadurch gekennzeichnet, daß er Zein G-200 enthält.

2. Antikaries-Kaugummi, enthaltend eine an sich bekannte Kaugummi-Grundmasse, Natriumfluorid, Calciumcitrat, Tricalcium-bis-Orthophosphat und Zusatzstoffe, nach Anspruch 1, dadurch gekennzeichnet, daß er Zein G-200 enthält, das derart in den Kaugummi eingebracht wird, daß zunächst Calciumcitrat und Tricalcium-bis-Orthophosphat mit der Grundmasse zu einer Grundmasse-Mischung vermischt werden, dann das Natriumfluorid mit Zein G-200 mittels eines Besprühverfahrens ummantelt, in eine saure wässrige Lösung eingebracht und in dieser einige Stunden gehalten wird, dann das so behandelte Natriumfluorid mit ätherischen Ölen zu einer Emulsion verarbeitet wird und schließlich diese Emulsion zusammen mit den üblichen Zusatzstoffen mittels eines Homogenisators in die Grundmasse-Mischung eingebracht wird.

3. Antikaries-Kaugummi nach Anspruch 1 oder 2, dadurch gekennzeichnet,

-8-

daß das Natriumfluorid mit 0,0004 bis 0,001 Gew.% Zein G-200 ummantelt ist.

4. Antikaries-Kaugummi nach einem der Ansprüche 1 bis 3, dadurch gekennzeichnet, daß das mit Zein G-200 ummantelte NaF in eine mit Zitronensäure auf pH 1,5 bis 1,8 eingestellte wässrige Lösung eingebracht und in dieser bei einer Temperatur von 25°C bis 30°C einige Stunden gehalten wird.

**Europäisches Patentamt**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | US-A-2 489 147 (B.N. LOUGOVOY)<br>* Seite 1 *<br>--- | 1 | A 23 G   3/30<br>A 61 K   7/16 |
| Y | US-A-2 154 482 (H.M. WEBER)<br>* Seite 1, linke Spalte, Zeilen 37-45; rechte Spalte, Zeilen 34-38; Seite 2, linke Spalte, Zeilen 1-21; Beispiel 1 *<br>--- | 1 | |
| Y | FR-A-1 228 510 (GENERAL FOODS CORP.)<br>* Zusammenfassung 1; Seite 1, rechte Spalte, Absatz 2; Seite 4, linke Spalte, Absatz 3; Seite 5, linke Spalte, Absatz 4; Seite 8, linke Spalte, Absatz 5; Beispiel 3 *<br>--- | 1,2 | |
| Y | FR-A-2 202 697 (THE PROCTER & GAMBLE COMP.)<br>* Seite 7, Zeile 17; Beispiel V *<br>--- | 1 | RECHERCHIERTE SACHGEBIETE (Int. Cl. ³)<br>A 23 G   3/00 |
| Y | US-A-1 887 930 (G.A. HATHERELL)<br>* Seite 3, Zeilen 47-59; Seite 2, Zeilen 49-53 *<br>--- | 1 | |
| Y | US-A-2 469 861 (W.P. COHOE)<br>* Spalte 1, Zeilen 31, 40-45; Spalte 2, Zeilen 1-3, 35-55; Beispiele 1,2 *<br>---                 -/- | 1,2 | |

Der vorliegende Recherchenbericht wurde für alle Patentansprüche erstellt.

| Recherchenort<br>DEN HAAG | Abschlußdatum der Recherche<br>16-06-1983 | Prüfer<br>GUYON R.H. |
|---|---|---|

**Europäisches Patentamt**

## EUROPÄISCHER RECHERCHENBERICHT

| | EINSCHLÄGIGE DOKUMENTE | | Seite 2 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| Y | US-A-4 284 650 (J.J. GOUPIL) * Ansprüche 1,5; Spalte 3, Zeilen 50-57 * | 1 | |

-----

RECHERCHIERTE SACHGEBIETE (Int. Cl. 3)

Der vorliegende Recherchenbericht wurde für alle Patentansprüche erstellt.

| Recherchenort DEN HAAG | Abschlußdatum der Recherche 16-06-1983 | Prüfer GUYON R.H. |
|---|---|---|